# EUROPÄISCHE PATENTSCHRIFT

(11) **EP 1 417 941 B1**
(45) Veröffentlichungstag und Bekanntmachung des Hinweises auf die Patenterteilung: **06.02.2008**
(21) Anmeldenummer: 02024596.5
(22) Anmeldetag: 05.11.2002
(51) Int. Cl.: A61F 2/46, A61F 2/36

(54) **Verfahren und Vorrichtung zum Registrieren eines Femur-Implantats**
Method and device for registering a femoral implant
Procédé et dispositif pour l'enregistrement d'un implant fémoral

(43) Veröffentlichungstag der Anmeldung: 12.05.2004
(73) Patentinhaber: BrainLAB AG, 85551 Kirchheim/Heimstetten (DE)
(72) Erfinder: Tuma, Gregor, 81675 München (DE); Schubert, Mario, 85652 Landsham (DE); Drumm, Peter, 81543 München (DE); Schaffrath, Claus, 81377 München (DE)
(74) Vertreter: Schwabe - Sandmair - Marx

(56) Entgegenhaltungen:
- WO-A-01/64143
- WO-A-02/071987
- WO-A-02/080824
- DE-A- 10 137 655
- US-A- 5 888 245
- US-B1- 6 370 418

## Beschreibung

Die vorliegende Erfindung bezieht sich auf Verfahren und eine Vorrichtung zum Registrieren und/oder Navigieren und/oder Positionieren eines Femur-Implantats.

Allgemein bezieht sich die Erfindung auf das Gebiet der Vorbereitung, Durchführung und Verifikation einer Implantation von künstlichen Gelenkkomponenten, insbesondere auf die Vorbereitung einer computerunterstützten Implantation eines Femur-Implantats während einer Hüftoperation. Es sind künstliche Hüftgelenke bekannt, wobei ein Femur-Implantat in einen körpereigenen Oberschenkelknochen (Femur) nach Entfernung eines körpereigenen Gelenkkopfes eingebracht und im Knochen verankert oder befestigt wird. Ein auf das Femur-Implantat aufsteckbarer oder mit dem Femur-Implantat fest verbundener sphärischer oder teilsphärischer Kopf kann von einem Wiederlager, wie zum Beispiel einer Gelenkpfanne, kugelgelenkartig aufgenommen werden.

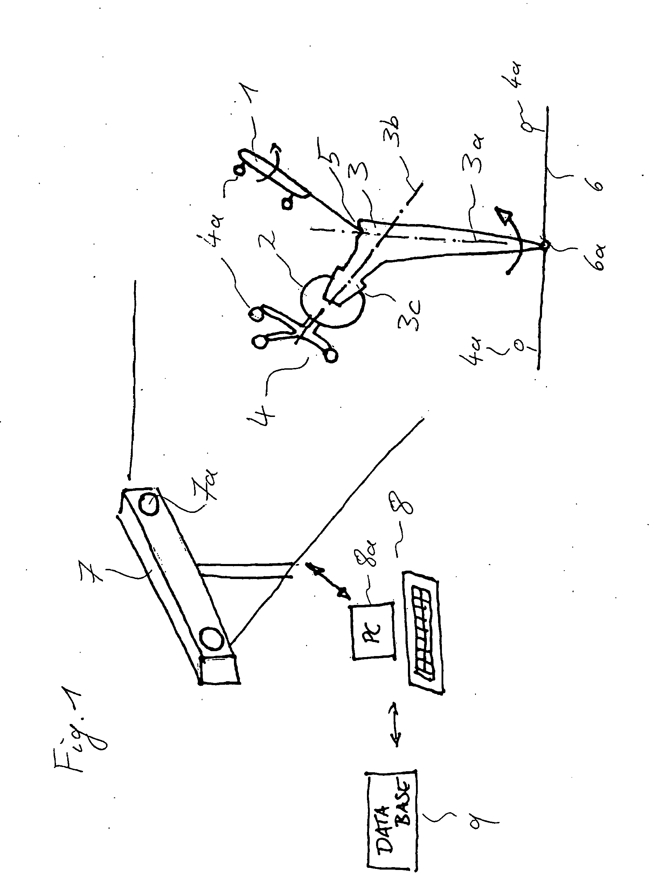

Wie in Figur 1 gezeigt, weist ein erfindungsgemäß verwendbares Femur-Implantat 3 einen zum Beispiel aus Titan oder Kunststoff bestehenden und sich z. B. konisch nach unten verjüngenden Schaft 3a auf, welcher in einen Oberschenkelknochen eingebracht werden kann, nachdem der körpereigene Gelenkkopf entfernt wurde. Der Schaft 3a geht in den Hals 3b über und auf der Vorderseite des Halses 3b ist ein Gelenk- oder Femur-Kopf 2 vorgesehen. Die räumliche Lage des Femur-Implantats kann zum Beispiel durch die Bestimmung der Positionen der Hals- und der Schaftachse definiert werden. Allgemein können auch anders aufgebaute und z. B modulare Femur-Implantate verwendet werden.

Der genaue Sitz des Femur-Implantats 3 im Oberschenkelknochen ist ein entscheidendes Kriterium für den Erfolg einer Hüftgelenkimplantation, wobei schon kleinste Abweichungen zu einem höheren Verschleiß und damit einer geringeren Lebensdauer des Hüftgelenkimplantats führen. Ist das Femur-Implantat nicht genau im Oberschenkelknochen positioniert, so kann es leicht zu einer Dislokation des in der acetabularen Komponente oder Pfanne angeordneten Femur-Kopfes 2 des Femur-Implantats 3 kommen, insbesondere wenn plötzliche Belastungen oder Stöße auf das künstlich eingesetzte Hüftgelenk einwirken. Bei nicht genau positionierten Hüftgelenkkomponenten können plötzlich auftretende Belastungen leicht zum Zusammenstoßen des Femur-Kopfes 2 oder der Halsachse 3b des Femur-Implantats 3 mit entweder dem Rand einer zugeordneten Pfanne oder weichem Gewebe oder einer Knochenstruktur führen, welche das Implantat umgeben. Demzufolge ist das genaue Positionieren der einzelnen Komponenten eines künstlichen Hüftgelenks und insbesondere das genaue Positionieren des Femur-Implantats wesentlich für einen Operationserfolg und für eine lange Lebensdauer des gesamten Gelenks.

Aus der US 6,002,859 sind eine Vorrichtung und ein Verfahren zur Implantation von künstlichen Gelenkkomponenten bekannt. Es wird vorgeschlagen durch Simulation die Position des Implantats zu bestimmen und ein Tracking der Hüfte, der Hüftgelenkpfanne und des Femur-Implantats unter Verwendung eines optischen Systems durchzuführen. Dabei werden sogenannte Targets an Knochen und Werkzeugen zum Einsetzen der künstlichen Gelenkkomponenten angebracht, wobei in einem Ausführungsbeispiel das Einsetzen von künstlichen achsensymmetrischen Hüftgelenkkomponenten beschrieben wird.

Die US 6,370,418 B1 offenbart eine Vorrichtung und ein Verfahren zum Messen der Position eines Implantates, welches mit mindestens einem Knochen in einem Körper verbunden ist, wobei die Position des Implantates relativ zu dem mindestens einem Knochen in dem Körper unter Verwendung von kleinen Tantal-Kugeln bestimmt wird.

Aus der US 5,888,245 ist eine Vorrichtung zur Ausrichtung eines Implantates relativ zu einem Femur-Knochen bekannt.

Es ist eine Aufgabe der vorliegenden Erfindung ein Verfahren und eine Vorrichtung zum Registrieren eines asymmetrischen Femur-Implantats vorzuschlagen, welche es ermöglichen ein nicht-achsensymmetrisches Femur-Implantat genau an einer gewünschten Stelle in einem Oberschenkelknochen zu positionieren.

Diese Aufgabe wird durch die Gegenstände der unabhängigen Ansprüche gelöst. Vorteilhafte Ausführungsformen sind in den Unteransprüchen definiert.

Nach dem erfindungsgemäßen Verfahren wird ein asymmetrisches Femur-Implantat registriert, indem unmittelbar an dem asymmetrischen Femur-Implantat, zum Beispiel auf der verlängerten Hals- oder Schaftachse, ein Marker, wie zum Beispiel ein Referenzstern, angebracht wird, um die räumliche Lage oder Orientierung einer ersten Femur-Implantat-Achse, wie zum Beispiel der Halsachse oder der Schaftachse zu ermitteln. Da die räumliche Lage eines asymmetrischen Implantats durch die Bestimmung der Lage einer Achse noch nicht definiert ist, wird gemäß einem ersten Aspekt der Erfindung das Femur-Implantat dadurch registriert, dass mit einer Spitze eines Zeigerinstruments mit einem oder mehreren daran angebrachten Markern auf einen Referenzbereich oder eine Referenzstelle des Femur-Implantats, wie zum Beispiel eine im Femur-Implantat vorgesehene Vertiefung oder charakteristische Form gezeigt wird, so dass das asymmetrische Femur-Implantat durch die so erfolgte Bestimmung der zweiten Achse vollständig referenziert ist, um die räumliche Lage des Femur-Implantats genau zu definieren. D. h. die exakte räumliche Lage des Femur-Implantats einschließlich des Verlaufs von Hals- und Schaftachse kann nach der erfindungsgemäß durchgeführten Referenzierung ermittelt werden.

Gemäß einem zweiten Aspekt der Erfindung wird das asymmetrische Femur-Implantat referenziert, indem die Position mindestens eines unmittelbar am Femur-Implantat angebrachten Markers oder Referenzsternes ermittelt wird, um zum Beispiel die räumliche Orientierung der Halsachse oder der Schaftachse des Femur-Implantats zu ermitteln. Weiterhin wird ein Bereich des Femur-Implantats, wie zum Beispiel ein spitz zulaufender Endbereich des Schaftes des Femur-Implantats auf eine vorgegebene Stelle eines Referenzierungs- oder Eichelements aufgebracht oder eingesteckt, an welchem ebenfalls mindestens ein Marker oder Referenzstern angebracht ist, um die Lage der zweiten Achse zu ermitteln. Als Referenzierungselement kann zum Beispiel eine ebene Platte mit einer darin an einer bestimmten Stelle vorgesehenen Vertiefung verwendet werden. Der spitz zulaufende Endbereich des Schaftes kann dann in die Vertiefung des Referenzierungselements eingebracht und optional kann das Femur-Implantat relativ zum Referenzierungselement bewegt werden, so dass aus der bekannten räumlichen Position des Referenzierungselements und der teilweise durch mindestens einen Marker festgelegten räumlichen Position des Femur-Implantats unter der Annahme, dass das Implantat in die Vertiefung eingesteckt wurde, die vollständige räumliche Lage des Femur-Implantats ermittelt werden kann. Die Bewegung oder Verkippung bzw. Verschwenkung des Femur-Implantats relativ zum Referenzierungselement kann zur weiteren Präzisierung der Bestimmung der räumlichen Lage und damit zur vollständigen Registrierung des Femur-Implantats verwendet werden.

Ebenso kann die zweite Implantat-Achse ohne ein Eichelement ermittelt werden, indem die Position der Spitze des Implantats fixiert wird. Z. B. kann die Spitze des Implantats in eine beliebige ortsfeste Vertiefung eingebracht werden, wobei eine Bewegung des so an der Spitze festgelegten Implantats dazu führt, dass die mit dem Implantat verbundene aus einem oder mehreren Markern bestehende Referenzgeometrie ein Kugelsegment beschreibt. Aus diesem Kugelsegment kann der Mittelpunkt der Kugel berechnet werden, welcher mit der Spitze des Implantats übereinstimmt, wodurch das Implantat vollständig referenziert werden kann.

Nach einem weiteren Aspekt der vorliegenden Erfindung können mindestens zwei Marker oder Referenzsterne an dem asymmetrischen Femur-Implantat angebracht werden, um die räumliche Lage der Halsachse und der Schaftachse des Femur-Implantats zu ermitteln und damit das Femur-Implantat zu registrieren.

Bei allen oben beschriebenen Ausführungsformen des erfindungsgemäßen Verfahrens ist es vorteilhaft, wenn der mindestens eine Marker oder Referenzstern, welcher an dem Femur-Implantat angebracht wird, etwa oder genau an einer Verlängerung einer der die räumliche Lage des Femur-Implantats beschreibenden Achsen angebracht wird. Besonders vorteilhaft wird ein Referenzstern so an dem Femur-Implantat angebracht, dass ein aufgrund der Markergeometrie bekannter Referenzpunkt des Referenzsterns auf der Mittelachse einer der die Lage des Femur-Implantats bestimmenden Achsen liegt. Beispielsweise kann ein Marker oder ein Referenzstern in der Richtung einer Verlängerung der Halsachse des Femur-Implantats auf oder statt dem Femur-Kopf und/oder auf dem Femur-Implantat entlang einer Verlängerung der Schaftachse, wie zum Beispiel dem spitz zulaufenden Ende des Femur-Implantats oder dem dem spitz zulaufenden Ende gegenüberliegenden Ende des Femur-Implantats angebracht werden.

Die räumliche Lage eines erfindungsgemäß registrierten Femur-Implantats ist nach erfolgter Registrierung bekannt. Dieses kann dann zu einer gewünschten Stelle navigiert werden. Ist weiterhin die räumliche Lage des Oberschenkelknochens zum Beispiel durch einen mit dem Oberschenkelknochen verbundenen Referenzstern bekannt, so kann das erfindungsgemäß registrierte Femur-Implantat genau im Oberschenkelknochen positioniert werden.

Eine gewünschte oder richtige Position des Femur-Implantats kann zum Beispiel durch Simulation vorausberechnet werden, wie beispielsweise in der US 6,002,859 beschrieben.

Basierend auf bildgebenden Verfahren, wie zum Beispiel einer Computertomographie, kann die für einen Patienten individuell vorteilhafteste Position der Hüftgelenksendoprothese und damit des Femur-Implantats ermittelt werden.

Man unterscheidet im Allgemeinen zementfrei oder mit Zement in einem Knochen befestigte Implantate, wobei zementfrei angebrachte Implantate in einen aufgeschnittenen Markkanal des Oberschenkelknochens eingebracht und im Wesentlichen durch Presspassung in dem Oberschenkelknochen gehalten werden. Das erfindungsgemäße Verfahren kann vorteilhaft bei zementierten bzw. mit Zement in einem Oberschenkelknochen befestigten Femur-Implantaten eingesetzt werden, wobei bei zementierten bzw. Zement verwendenden Verfahren ein größeres Volumen als das des einzusetzenden Femur-Implantats aus dem Oberschenkelknochen in einem vorbereitenden Schritt herausgenommen wird und anschließend Zement und das Femur-Implantat in den Oberschenkelknochen eingesetzt wird, so dass das Femur-Implantat noch relativ zum Oberschenkelknochen bewegt und damit möglichst exakt positioniert werden kann, bevor der Zement aushärtet. Erfindungsgemäß registrierte und navigierte Femur-Implantate können bei zementierten Implantaten noch leicht exakt ausgerichtet und positioniert werden, wobei die erfindungsgemäße Registrierung und Navigation des Femur-Implantats im Vergleich zu bekannten Verfahren zu einer höheren Genauigkeit führt.

Da das Femur-Implantat durch mindestens einen unmittelbar an dem Femur-Implantat angebrachten Marker oder Referenzstern registriert und navigiert werden kann, ist die Genauigkeit bei der Positionsermittlung des Femur-Implantats größer im Vergleich zu Verfahren, wie sie zum Beispiel aus der US 6,002,859 bekannt sind, bei welchen Werkzeuge und Vorrichtungen zur Vorbereitung und zum Einsetzen des Implantats navigiert werden, da die Position des zu navigierenden Femur-Implantats gemäß dem Stand der Technik nur indirekt erfasst wird, so dass die Genauigkeit der Positionsermittlung eines Femur-Implantats mit dem erfindungsgemäßen Verfahren durch das direkte Bestimmen der Implantatposition verbessert werden kann.

Vorteilhaft kann dass erfindungsgemäße Verfahren auch zum Verifizieren der Position eines in einen Oberschenkelknochen eingesetzten Femur-Implantats verwendet werden, um zum Beispiel Parameter zu ermitteln, welche für die richtige Positionierung, den korrekten Sitz und damit die lange Lebensdauer eines Implantats wesentlich sind. Beispiele solcher Parameter sind: die Position des Drehmittelpunkts des Hüftgelenks; die Länge des Beines; die Anteversion des Femur-Kopfes; die Einsetztiefe des Femur-Implantats; Anterior/Posterior Shift, d.h. die Parallelverschiebung des Implantats bezüglich der Oberschenkelknochen-Schaft-Achse in Anterior - Posterior-Richtung (Bauch - Rücken); Anterior/Posterior Tilt, d.h. die Verdrehung des Implantats um den Schnittpunkt von Schaft- und Halsachse in A-P-Richtung; Lateral-Medial Shift, d.h. die Parallelverschiebung des Implantats bezüglich der Oberschenkelknochen-Schaft-Achse in Medial-Lateral-Richtung (links - rechts, bzw. Außen - Mitte); und Varus/Valgus Tilt, d.h. die Verdrehung des Implantats um den Schnittpunkt von Schaft- und Halsachse in Medial-Lateral-Richtung.

Gemäß einem weiteren Aspekt bezieht sich die Erfindung auf ein Computerprogramm, welches, wenn es in einen Computer geladen wird oder ist oder auf einem Computer läuft, einen oder mehrere der oben beschriebenen Verfahrensschritte ausführt. Weiterhin bezieht sich die Erfindung auf ein Speichermedium für ein solches Programm oder ein Computerprogrammprodukt mit einem solchen Programm.

Eine erfindungsgemäße Vorrichtung zum Registrieren und/oder Navigieren und/oder Positionieren eines asymmetrischen Femur-Implantats weist eine Erfassungsvorrichtung, wie zum Beispiel optische Kameras, zum Beispiel zur Erfassung von Infrarotstrahlen, oder ein auf Schallwellen oder Funkwellen basierendes System oder eine andere geeignete Vorrichtung zum Erfassen von Positionssignalen auf, welche zum Beispiel von Markern emittiert oder reflektiert werden. Weiterhin ist eine Datenbank vorgesehen, in welcher Daten zur Geometrie und/oder Abmessung eines oder mehrerer verschiedener Femur-Implantate gespeichert sind. Erfindungsgemäß ist eine Recheneinheit vorgesehen, welche aus den von der Erfassungsvorrichtung gelieferten Positionssignalen die räumliche Lage eines Femur-Implantats bestimmt, wobei eines oder mehrere der oben beschriebenen Verfahren zum Registrieren, Navigieren und/oder Positionieren eines Femur-Implantats verwendet werden können.

Ein asymmetrisch ausgebildetes Femur-Implantat hart mindestens einen Befestigungspunkt für mindestens einen Marker oder einen Referenzstern, wobei der mindestens eine Marker oder ein frei bestimmbarer und z. B. durch eine Software zu ermittelnder Ursprung des Referenzsterns bevorzugt auf einer Halsachse oder einer Schaftachse des Femur-Implantats angeordnet werden können.

Vorteilhaft ist der Endbereich des Femur-Implantats, an welchem ein zum Beispiel kugelförmiger oder teilkugelförmiger Femur-Kopf angebracht werden kann, so ausgebildet, dass mindestens ein Marker oder Referenzstern auf dem Endabschnitt der Halsachse oder alternativ auch auf dem Femur-Kopf befestigt werden kann. Bevorzugt ist die Verbindung zwischen Femur-Implantat und aufgesetztem Marker oder Referenzstern so ausgebildet, dass der mindestens eine Marker oder Referenzstern verdrehsicher auf dem Femur-Implantat angeordnet oder befestigt ist und vorteilhaft leicht wieder abgenommen werden kann.

Vorteilhaft sind der oder die Marker und/oder der oder die Referenzsterne, welche an dem Femur-Implantat angebracht werden, verdrehsicher bezüglich des Femur-Implantats gelagert. Dies kann zum Beispiel durch entsprechende Geometrien, wie zum Beispiel Nuten, in welche Verdrehsicherungen eingreifen, durch formschlüssige Verbindungen oder durch kraftschlüssige Verbindungen wie zum Beispiel verrutschsichere oder gummiartige Elemente, welche auf entsprechenden Gegenflächen aufliegen, realisiert werden. Allgemein ist es vorteilhaft einen oder mehrere Marker oder Referenzsterne so an dem Femur-Implantat anzubringen, dass sie nicht einfach, d. h. nur durch größere Kräfte als bei einer normalen Belastung auftretende Kräfte bewegt werden können.

Auf dem Implantat kann ein Referenzbereich, wie zum Beispiel eine Vertiefung oder eine charakteristische Oberflächenform und/oder Oberflächenstruktur vorgesehen sein, auf welche mit einem Zeigerinstrument gezeigt oder in welche ein Zeigerinstrument eingebracht oder angelegt werden kann, um das Femur-Implantat erfindungsgemäß registrieren zu können.

Ein System kann aus einem wie oben beschriebenen Femur-Implantat und einem Referenzierungselement, wie zum Beispiel einem Zeigerinstrument, welches an eine oder mehrere vorgegebene oder frei wählbare Stellen des Femur-Implantats angelegt werden kann, bestehen oder kann eine Referenzierungsvorrichtung, wie zum Beispiel eine mit mindestens einem Marker oder Referenzstern versehene plattenförmige Vorrichtung mit einem vorgegebenen Kontaktpunkt, beispielsweise einer Vertiefung, in welche ein bestimmter Bereich des Femur-Implantats, wie zum Beispiel ein äußeres Ende der Schaftachse eingesetzt und zum Beispiel relativ zur Referenzierungsvorrichtung bewegt oder geschwenkt werden kann, aufweisen.

Die Erfindung wird nachfolgend anhand eines Ausführungsbeispiels beschrieben werden. Es zeigen:
- Figur 1: ein Femur-Implantat mit erfindungsgemäßen Registrierungselementen.
- Figur 2: ein in einen Knochen eingesetztes Femur-Implantat

Figur 1 zeigt schematisch ein Femur-Implantat 3, welches einen Schaft 3a und einen in einem Winkel α dazu angeordneten Hals 3b aufweist, wobei die Position des Schafts 3a durch die strichpunktiert eingezeichnete Schaftachse und die Position des Halses 3b durch die strichpunktiert eingezeichnete Halsachse definiert ist. Der Hals 3b weist an seinem vorderen Ende ein kegelstumpfförmiges Element 3c auf, auf welchem entweder ein etwa kugelförmiger Femur-Kopf 2 oder ein Adapterelement zum Anbringen eines Referenzsterns 4 mit darauf angeordneten Markern 4a bevorzugt verdrehsicher zum Registrieren, Navigieren und/oder Positionieren des Implantats 3 befestigt werden kann.

Das Femur-Implantat 3 kann erfindungsgemäß registriert werden, indem die Halsachse durch die Position des auf dem Hals 3b aufgebrachten Referenzsternes 4 ermittelt wird. Die Position des Schaftes 3a kann gemäß einer ersten Ausführungsform mit einem Zeigerinstrument 1 bestimmt werden, an welchem Marker 4a angebracht sind, wobei das Zeigerinstrument 1 auf einen vorgegebenen Referenzbereich oder eine Vertiefung 5 des Femur-Implantats 3 gebracht und zum Beispiel wie durch den Pfeil gezeigt bewegt wird, um die räumliche Lage des Referenzbereiches 5 und damit die Lage der Schaftachse zu ermitteln.

Gemäß einer weiteren Ausführungsform kann das Femur-Implantat 3 registriert werden, indem ein bestimmter Bereich des Femur-Implantats 3, wie zum Beispiel das spitz zulaufende Ende des Schaftes 3a auf oder in einen Referenzbereich 6a oder eine Referenzstelle eines Referenzierungselements 6 auf- oder eingebracht und dort gekippt oder geschwenkt wird, wie durch den Pfeil in Figur 1 gezeigt, um somit die Orientierung der Schaftachse zu ermitteln.

Alternativ oder ergänzend können auch weitere Marker oder Referenzsterne an dem Femur-Implantat 3 angebracht werden, wobei zu beachten ist, dass Femur-Implantate nicht beliebig verändert werden können, da diese speziellen Zulassungsverfahren unterliegen.

Figur 1 zeigt weiter eine Erfassungsvorrichtung 7 mit zwei IR-Kameras 7a, um die von den Markern 4a ausgesandten und/oder reflektierten Signale zu erfassen. Die von der Erfassungsvorrichtung 7 erfassten Positionssignale der Marker 4a werden an eine Recheneinheit 8 mit zugeordneter Datenbank 9 übertragen, um die Position des Femur-Implantats 3 zu ermitteln, so dass das Femur-Implantat 3 registriert ist und navigiert oder an einer bestimmten Stelle positioniert werden kann. Auf einem Bildschirm 8a können zum Beispiel Positionsinformationen des Implantats 3 dargestellt werden.

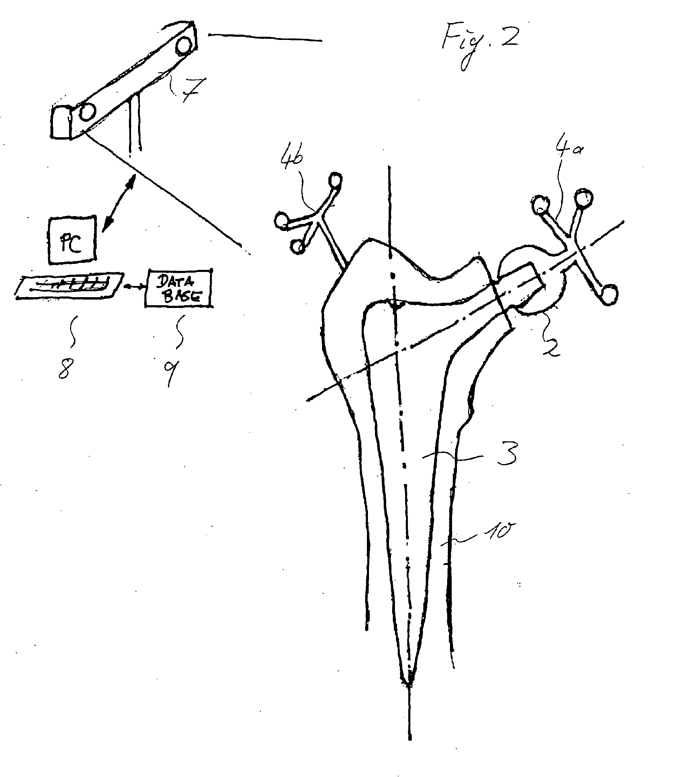

Figur 2 zeigt das in einen Oberschenkelknochen 10 eingesetzte Implantat 3, welches im Knochen 10, dessen Position durch den Referenzstern 4b ermittelt wird, durch Verwendung der über den mit dem Implantat 3 verbundenen Referenzstern 4a gewonnenen Positionsdaten des Implantats 3 genau positioniert werden kann.

## Patentansprüche

1. Verfahren zur Vorbereitung einer Implantation eines asymmetrischen Femur-Implantats (3), wobei das asymmetrische Femur-Implantat (3) einen Schaft (3a) und einem Hals (3b) aufweist und wobei die Position einer Schaftachse und einer Halsachse des asymmetrischen Femur-Implantats (3) ermittelt wird.

2. Verfahren nach Anspruch 1, wobei die Position der Schaftachse und/oder die Position der Halsachse durch mindestens einen Marker (4a) oder einen Referenzstern (4) ermittelt wird, welcher an dem Femur-Implantat (3) angebracht ist.

3. Verfahren nach einem der vorhergehenden Ansprüche, wobei die räumliche Position der Schaftachse und/oder der Halsachse mit einem Zeigerinstrument (1) ermittelt wird.

4. Verfahren nach einem der vorhergehenden Ansprüche, wobei die räumliche Position der Schaftachse und/oder der Halsachse mit einem Referenzierungselement (6) ermittelt wird, auf welches das Femur-Implantat (3) gesetzt werden kann.

5. Verfahren nach einem der beiden vorhergehenden Ansprüche, wobei an dem Zeigerinstrument (1) und/oder an dem Referenzierungselement (6) mindestens ein Marker (4a) oder ein Referenzstern (4) angebracht ist.

6. Computerprogramm, welches, wenn es in einen Computer geladen wird oder auf einem Computer läuft, die Position einer Schaftachse und einer Halsachse eines asymmetrischen Femur-Implantats (3) unter Verwendung von Daten aus einer Datenbank (9), in welcher die Geometrie des Femur-Implantats (3) gespeichert ist, und unter Verwendung von Daten einer Erfassungsvorrichtung (7) zum Erfassen der räumlichen Lage mindestens eines Markers (4a), welcher mit dem asymmetrischen Femur-Implantat (3) verbunden ist, ermittelt.

7. Programmspeichermedium oder Computerprogrammprodukt mit dem Programm nach dem vorhergehenden Anspruch.

8. Vorrichtung zum Registrieren eines mit mindestens einem Marker (4a) verbundenen asymmetrischen Femur-Implantats (3) mit einer Erfassungsvorrichtung (7) zum Erfassen der räumlichen Lage des mindestens einen Markers (4a); einer Datenbank (9), in welcher die Geometrie des Femur-Implantats (3) gespeichert ist; und einer Recheneinheit (8), mit welcher die Position einer Schaftachse und einer Halsachse des asymmetrischen Femur-Implantats (3) ermittelt wird, wobei der mindestens eine Marker als Referenzstern (4) ausgebildet ist.

## Claims

1. A method for preparing an implantation of an asymmetrical femoral implant (3) comprising a shaft (3a) and a collar (3b), wherein the position of a shaft axis and a collar axis of the asymmetrical femoral implant (3) is ascertained.

2. The method according to claim 1, wherein the position of the shaft axis and/or the position of the collar axis is ascertained using at least one marker (4a) or reference star (4) attached to the femoral implant (3).

3. The method according to any one of the preceding claims, wherein the spatial position of the shaft axis and/or the collar axis is ascertained using a pointer instrument (1).

4. The method according to any one of the preceding claims, wherein the spatial position of the shaft axis and/or the collar axis is ascertained using a referencing element (6) onto which the femoral implant (3) can be set.

5. The method according to any one of the preceding two claims, wherein at least one marker (4a) or reference star (4) is attached to the pointer instrument (1) and/or to the referencing element (6).

6. A computer program which, when it is loaded onto a computer or is running on a computer, ascertains the position of a shaft axis and a collar axis of an asymmetrical femoral implant (3) using data from a database (9) in which the geometry of the femoral implant (3) is stored, and using data of a detection device (7) for detecting the spatial position of at least one marker (4a) connected to the asymmetrical femoral implant (3).

7. A program storage medium or computer program product comprising the program according to the preceding claim.

8. A device for registering an asymmetrical femoral implant (3) connected to at least one marker (4a), comprising: a detection device (7) for detecting the spatial position of the at least one marker (4a); a database (9) in which the geometry of the femoral implant (3) is stored; and a computational unit (8) using which the position of a shaft axis and a collar axis of the asymmetrical femoral implant (3) is ascertained, wherein the at least one marker is formed as a reference star (4).

## Revendications

1. Procédé pour préparer l'implantation d'un implant fémoral asymétrique (3), dans lequel l'implant fémoral asymétrique (3) comporte une tige (3a) et un col (3b), et dans lequel on détermine la position d'un axe de tige et d'un axe de col de l'implant fémoral asymétrique (3).

2. Procédé selon la revendication 1, dans lequel on détermine la position de l'axe de tige et/ou la position de l'axe de col par au moins un repère (4a) ou une étoile de référence (4) qui est placée sur l'implant fémoral (3).

3. Procédé selon l'une quelconque des revendications précédentes, dans lequel on détermine la position spatiale de l'axe de tige et/ou de l'axe de col avec un instrument à aiguille (1).

4. Procédé selon l'une quelconque des revendications précédentes, dans lequel on détermine la position spatiale de l'axe de tige et/ou de l'axe de col avec un élément de repérage (6) sur lequel on peut placer l'implant fémoral (3).

5. Procédé selon l'une quelconque des deux revendications précédentes, dans lequel au moins un repère (4a) ou une étoile de référence (4) est placée sur l'instrument à aiguille (1) et/ou sur l'élément de repérage (6).

6. Programme d'ordinateur qui, lorsqu'il est chargé dans un ordinateur ou tourne sur un ordinateur, détermine la position d'un axe de tige et d'un axe de col d'un implant fémoral asymétrique (3), en utilisant des données provenant d'une banque de données dans laquelle est mémorisée la géométrie de l'implant fémoral (3), et en utilisant des données d'un dispositif de relevé (7) pour relever la position spatiale d'au moins un repère (4a) qui est relié à l'implant fémoral asymétrique (3).

7. Support de mémoire de programme ou produit de programme d'ordinateur avec le programme selon la revendication précédente.

8. Dispositif pour repérer un implant fémoral asymétrique (3) relié à au moins un repère (4a), avec un dispositif de relevé (7) pour relever la position spatiale du au moins un repère (4a) ; une banque de données (9) dans laquelle est mémorisée la géométrie de l'implant fémoral (3); et une unité informatique (8) avec laquelle on détermine la position d'un axe de tige et d'un axe de col de l'implant fémoral asymétrique (3), le au moins un repère étant réalisé sous la forme d'une étoile de référence (4).
